# EUROPEAN PATENT SPECIFICATION

(11) **EP 3 841 950 B1**
(45) Date of publication and mention of the grant of the patent: **06.03.2024**
(21) Application number: 19852548.7
(22) Date of filing: 09.07.2019
(51) Int. Cl.: A61B 1/00, A61B 1/045, G02B 23/24, A61B 5/06, A61B 34/20

(54) **ENDOSCOPIC SYSTEM AND POSITION DERIVING METHOD**
ENDOSKOPISCHES SYSTEM UND VERFAHREN ZUR POSITIONSABLEITUNG
SYSTÈME ENDOSCOPIQUE ET PROCÉDÉ DE DÉDUCTION DE POSITIONS

(30) Priority: 23.08.2018 JP 2018156077
(43) Date of publication of application: 30.06.2021
(73) Proprietor: FUJIFILM Corporation, Tokyo 106-8620 (JP)
(72) Inventor: IMAMURA, Kaori, Ashigarakami-gun, Kanagawa 258-8538 (JP); SHIMIZU, Takaaki, Ashigarakami-gun, Kanagawa 258-8538 (JP)
(74) Representative: Kudlek, Franz Thomas
(86) International application number: PCT/JP2019/027226
(87) International publication number: WO 2020/039776

(56) References cited:
- WO-A1-2011/155383
- WO-A1-2017/217162
- JP-A- H10 507 104
- JP-A- 2003 290 129
- JP-A- 2003 524 443
- JP-A- 2007 330 348
- JP-A- 2008 011 913
- US-A1- 2016 100 772

## Description

### BACKGROUND OF THE INVENTION

### 1. Field of the Invention

The present disclosure relates to an endoscope system and a position derivation method.

### 2. Description of the Related Art

In the related art, in an inspection inside a subject using an endoscope (hereinafter, referred to as an "endoscope inspection"), supporting the endoscope inspection has been performed by detecting a shape of an insertion part of the endoscope inserted into the subject and displaying a shape image indicating the detected shape of the insertion part on a display unit. Further, a plurality of magnetic field generating elements and a plurality of magnetic field detecting elements are used in detecting the shape of the insertion part of the endoscope.

For example, JP2006-136413A discloses that a technique of calculating the shape of the insertion part of the endoscope by using frequency components corresponding to frequencies of drive signals for generating magnetic fields from the magnetic field generating elements from detection signals by the plurality of magnetic field detecting elements.

An endoscope system according to the preamble of claim 1 is known from WO 2017/217162 A1. Further relevant prior art is known from US 2016/0100772 A1 and JP 2008011913 A.

### SUMMARY OF THE INVENTION

In a case where a shape of an insertion part of an endoscope is calculated by using a plurality of magnetic field generating elements and a plurality of magnetic field detecting elements, a generation device that causes the magnetic field generating elements to generate magnetic fields notifies the following timing to a detection device that detects detection signals by the magnetic field detecting elements. That is, in this case, the generation device notifies the timing, at which the magnetic field generating elements are caused to generate magnetic fields, to the detection device. The detection device acquires signals detected by the magnetic field detecting elements on the basis of the notified timing.

In a case where the notification of the timing is performed by wired communication, for example, a cable that connects the generation device and the detection device often straddles the bed on which the subject lies. Therefore, an inspector who operates the endoscope is careful about the handling of the cable, which reduces usability.

On the other hand, it is conceivable to perform the notification of the timing by wireless communication according to a predetermined communication standard such as a wireless local area network (LAN) or Bluetooth (registered trademark). However, in this case, a wireless communication unit for notifying the timing at which the magnetic field is generated is necessary. Further, in this case, a process such as authentication between devices performing the wireless communication is also necessary.

The present disclosure is made in view of such circumstances, and an object thereof is to provide an endoscope system and a position derivation method which can wirelessly connect a device that generates a magnetic field and a device that detects a magnetic field without being provided with a wireless communication unit for notifying a timing at which the magnetic field is generated.

In order to achieve the above object, an endoscope system according to claim 1 is provided. Advantageous embodiments are the subject matter of the dependent claims.

The invention further provides a position derivation method comprising the features of claim 15.

According to the present disclosure, a device that generates a magnetic field and a device that detects a magnetic field can be wirelessly connected without providing a wireless communication unit for notifying a timing at which the magnetic field is generated.

### BRIEF DESCRIPTION OF THE DRAWINGS

Fig. 1 is a configuration diagram illustrating an example of a configuration of an endoscope system according to each embodiment.
Fig. 2 is a block diagram illustrating an example of the configuration of the endoscope system according to each embodiment.
Fig. 3 is a configuration diagram illustrating an example of a reception coil unit and a transmission coil unit according to each embodiment.
Fig. 4 is a block diagram illustrating an example of a functional configuration of a transmission controller according to a first embodiment.
Fig. 5 is a diagram for describing a first waveform and a second waveform according to the first embodiment.
Fig. 6 is a block diagram illustrating an example of a functional configuration of an overall controller according to the first embodiment.
Fig. 7 is a block diagram illustrating an example of a hardware configuration of the transmission controller according to each embodiment.
Fig. 8 is a block diagram illustrating an example of a hardware configuration of the overall controller according to each embodiment.
Fig. 9 is a flowchart illustrating an example of a magnetic field generation process according to each embodiment.
Fig. 10 is a flowchart illustrating an example of a position derivation process according to the first embodiment.
Fig. 11 is a diagram for describing a first waveform and a second waveform according to a modification example.
Fig. 12 is a diagram for describing a first waveform and a second waveform according to a modification example.
Fig. 13 is a block diagram illustrating an example of a functional configuration of a transmission controller according to a second embodiment.
Fig. 14 is a diagram for describing a first waveform and a second waveform according to the second embodiment.
Fig. 15 is a block diagram illustrating an example of a functional configuration of an overall controller according to the second embodiment.
Fig. 16 is a flowchart illustrating an example of a position derivation process according to the second embodiment.
Fig. 17 is a diagram for describing a first waveform and a second waveform according to a modification example.

### DESCRIPTION OF THE PREFERRED EMBODIMENTS

Hereinafter, embodiments for implementing the technique of the present disclosure will be described in detail with reference to the drawings.

### [First Embodiment]

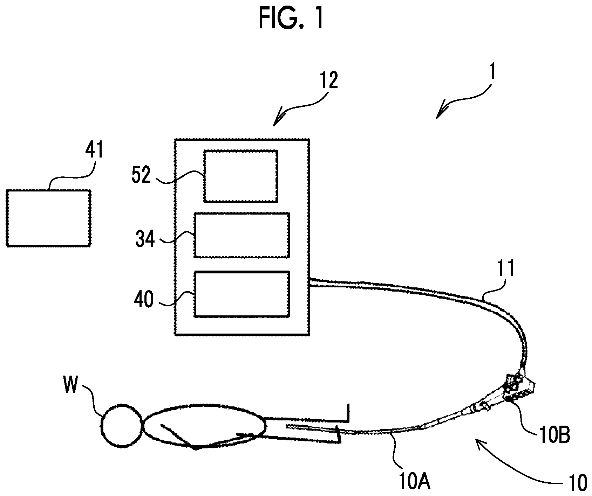

First, the configuration of an endoscope system 1 according to the embodiment will be described with reference to Fig. 1. As illustrated in Fig. 1, the endoscope system 1 comprises an endoscope 10 which captures an image (hereinafter, referred to as an "endoscopic image") of an inside of a subject W, and an endoscope inspection device 12.

The endoscope 10 comprises an insertion part 10A and an operation part 10B, and in a case where an endoscope inspection is performed, the inspector operates the operation part 10B to insert the insertion part 10A into the subject W and to capture an endoscopic image of the inside of the subject W. The endoscope inspection device 12 connected to the endoscope 10 by a cable 11 comprises a video processor 34, an overall controller 40, a transmission unit 41, and a display unit 52 such as a liquid crystal display. The video processor 34 controls capturing of the endoscopic image by the endoscope 10. The overall controller 40 controls the entire endoscope system 1.

The video processor 34, the overall controller 40, and the display unit 52 are installed on the same installation table, and are connected to each other by a cable so as to be communicable. The transmission unit 41 is installed apart from the other units of the endoscope inspection device 12, and is connected to the power supply by a cable, but is not connected to the other units of the endoscope inspection device 12 by a cable. That is, the inspector can move the transmission unit 41 independently from the other units of the endoscope inspection device 12.

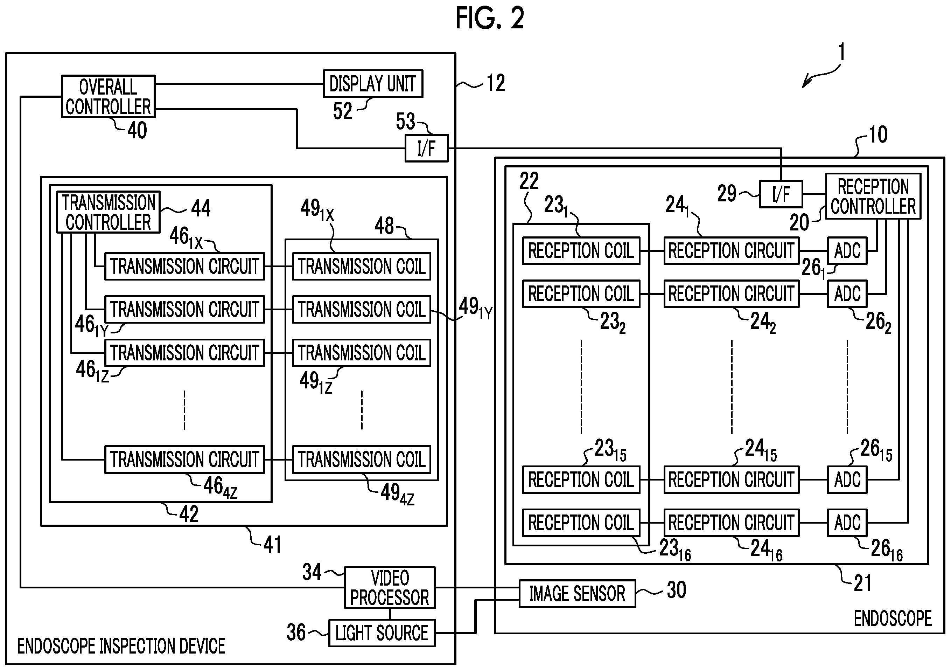

Next, the detailed configuration of the endoscope 10 and the endoscope inspection device 12 will be described with reference to Fig. 2. As illustrated in Fig. 2, the endoscope 10 comprises an image sensor 30 including imaging elements, such as a charge coupled device (CCD) image sensor, and a complementary metal oxide semiconductor (CMOS) image sensor. The endoscope 10 transmits light emitted from a light source 36 through a transmission path (not illustrated), emits the light from an emission unit (not illustrated) provided on a distal end of the insertion part 10A, and illuminate the inside of the subject W with the emitted light, under the control of the video processor 34. A reflected light from the subject W by the illumination light is imaged on the image sensor 30 by an objective lens (not illustrated), and an image signal according to the endoscopic image as the formed optical image is output to the video processor 34 of the endoscope inspection device 12 via the cable 11. A predetermined image process is performed on the input image signal by the video processor 34, and image data of the endoscopic image obtained by the image process is output to the overall controller 40.

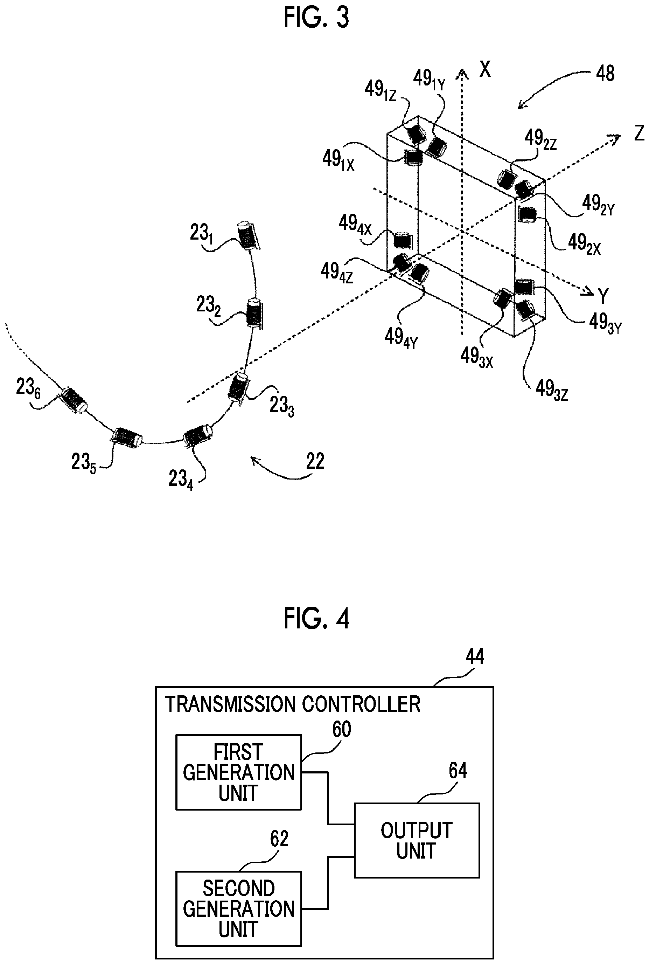

In addition, the transmission unit 41 comprises a transmission control unit 42 and a transmission coil unit 48. As illustrated in Fig. 3, the transmission coil unit 48 comprises a plurality of (in the embodiment, 12) transmission coils 49_{1X}, 49_{1Y}, 49_{1Z}, 49_{2X}, 49_{2Y}, 49_{2Z}, 49_{3X}, 49_{3Y}, 49_{3Z}, 49_{4X}, 49_{4Y}, and 49_{4Z}. As illustrated in Fig. 3, the transmission coils 49 of the embodiment are grouped together into combinations of three transmission coils of which the axes respectively face the X-axis, Y-axis, and Z-axis directions, so that the transmission coil unit 48 comprises four transmission coil groups. In the embodiment, the transmission coils 49 are generically referred to as the "transmission coil 49", and each transmission coil group is generically referred by adding numerical sign (₁ ... ₄) with the alphabet omitted after the "transmission coil 49". Further, in the embodiment, in a case of individually distinguishing the transmission coils 49, signs (_{1X} ... _{4Z}) indicating individuals are added after the "transmission coil 49". Each of the transmission coils 49 is an example of the magnetic field generating elements.

Specifically, the transmission coil unit 48 comprises a set of the transmission coil 49_{1X} oriented in the X-axis direction, the transmission coil 49_{1Y} oriented in the Y-axis direction, and the transmission coil 49_{1Z} oriented in the Z-axis direction, and a set of the transmission coil 49_{2X} oriented in the X-axis direction, the transmission coil 49_{2Y} oriented in the Y-axis direction, and the transmission coil 49_{2Z} oriented in the Z-axis direction. Further, the transmission coil unit 48 comprises a set of the transmission coil 49_{3X} oriented in the X-axis direction, the transmission coil 49_{3Y} oriented in the Y-axis direction, and the transmission coil 49_{3Z} oriented in the Z-axis direction, and a set of the transmission coil 49_{4X} oriented in the X-axis direction, the transmission coil 49_{4Y} oriented in the Y-axis direction, and the transmission coil 49_{4Z} oriented in the Z-axis direction. In this manner, the transmission coil unit 48 of the embodiment is equivalent to a state of comprising four sets of 3-axis coils as the transmission coils 49.

The transmission control unit 42 comprises a transmission controller 44, and transmission circuits 46_{1X}, 46_{1Y}, 46_{1Z}, 46_{2X}, 46_{2Y}, 46_{2Z}, 46_{3X}, 46_{3Y}, 46_{3Z}, 46_{4X}, 46_{4Y}, and 46_{4Z} connected to the transmission coils 49 on a one-to-one basis. In the embodiment, similar to the transmission coils 49, the transmission circuits 46 are generically referred to as the "transmission circuit 46", and each transmission circuit group is generically referred by adding numerical sign (₁ ... ₄) with the alphabet omitted after the "transmission circuit 46". Further, in the embodiment, similar to the transmission coils 49, in a case of individually distinguishing the transmission circuits 46, signs (_{1X} ... _{4Z}) indicating individuals are added after the "transmission circuit 46".

The transmission controller 44 generates a first waveform (hereinafter, simply referred to as a "first waveform") for detecting the position of the insertion part 10A of the endoscope 10 to be inserted into the subject W. Further, the transmission controller 44 generates a second waveform (hereinafter, simply referred to as a "second waveform") for detecting a synchronization timing, which is different from the first waveform and can be distinguished from the first waveform. The transmission controller 44 outputs each waveform to the transmission circuits 46. The transmission controller 44 will be described below in detail.

Each transmission circuit 46 drives the transmission coil 49 connected thereto, according to the waveform input from the transmission controller 44. Specifically, each transmission circuit 46 outputs the drive signal according to the waveform input from the transmission controller 44 to the transmission coil 49 connected thereto, and thereby drives the transmission coil 49 connected thereto. The transmission circuit 46 is an example of a drive unit. Each transmission coil 49 generates a magnetic field according to the drive signal input from the transmission circuit 46 connected thereto.

On the other hand, a reception unit 21 provided inside the insertion part 10A of the endoscope 10 comprises a reception controller 20, a reception coil unit 22, reception circuits 24 (24₁ to 24₁₆), analog-to-digital converts (ADC) 26 (26₁ to 26₁₆), and an interface (I/F) 29. The reception controller 20 controls the entire reception unit 21.

As illustrated in Fig. 3, the reception coil unit 22 comprises 16 reception coils 23₁ to 23₁₆ (six reception coils are illustrated in the example of Fig. 3), as an example. In the embodiment, similar to the transmission coils 49, the reception coils 23, the reception circuits 24, and the ADCs 26 are generically referred to as the "reception coil 23", the "reception circuit 24", and the "ADC 26", respectively. Further, in the embodiment, similar to the transmission coils 49, in a case of individually distinguishing the reception coils 23, the reception circuits 24, and the ADCs 26, signs (₁ ... ₁₆) indicating individuals are added after the "reception coil 23 ", the "reception circuit 24", and the "ADC 26".

The reception coils 23 are arranged in the insertion part 10A of the endoscope 10 along a direction of being inserted into the subject W. The reception coils 23 detect the magnetic fields generated by the transmission coils 49 of the transmission coil unit 48. Each reception coil 23 is connected to the reception circuit 24, and outputs a signal (hereinafter, referred to as a "detection signal") according to the detected magnetic field to the reception circuit 24. Each of the reception coils 23 is an example of the magnetic field detecting elements. The reception circuit 24 includes a low pass filter (LPF) and an amplifier (neither is illustrated), and outputs the detection signal which is amplified by the amplifier after the removal of the disturbance noise by the LPF, to the ADC 26. The ADC 26 converts the input analog detection signal into a digital detection signal, and outputs the digital detection signal to the reception controller 20. The reception controller 20 transmits the detection signal input from each ADC 26 to the endoscope inspection device 12 via the I/F 29.

The detection signal transmitted from the reception unit 21 of the endoscope 10 to the endoscope inspection device 12 is input to the overall controller 40 via an I/F 53.

The overall controller 40 derives the position and orientation (posture) of each reception coil 23 on the basis of the input detection signal. Further, the overall controller 40 derives the shape of the insertion part 10A of the endoscope 10 on the basis of the derived position and orientation of each reception coil 23. The overall controller 40 will be described below in detail.

Next, the functional configuration of the transmission controller 44 according to the embodiment will be described with reference to Fig. 4.

As illustrated in Fig. 4, the transmission controller 44 includes a first generation unit 60, a second generation unit 62, and an output unit 64. The first generation unit 60 generates the first waveform. The second generation unit 62 generates the second waveform. In the embodiment, the first waveform and the second waveform have different waveform shapes, so that the first waveform and the second waveform can be distinguished from each other. As long as the first waveform and the second waveform can be distinguished from each other, the first waveform and the second waveform may have the same waveform shape. For example, a form in which the first waveform and the second waveform have the same waveform shape but have different frequencies, amplitudes, or phases is exemplified. The first waveform and the second waveform can be distinguished from each other by differentiating at least one of the frequencies, phases, amplitudes, or waveform shapes of the first waveform and second waveform.

The output unit 64 outputs the second waveform to the transmission circuits 46, and then outputs the first waveform to the transmission circuits 46. As a result, the transmission circuits 46 drive the transmission coils 49 with the second waveform, and then drive the transmission coils 49 with the first waveform.

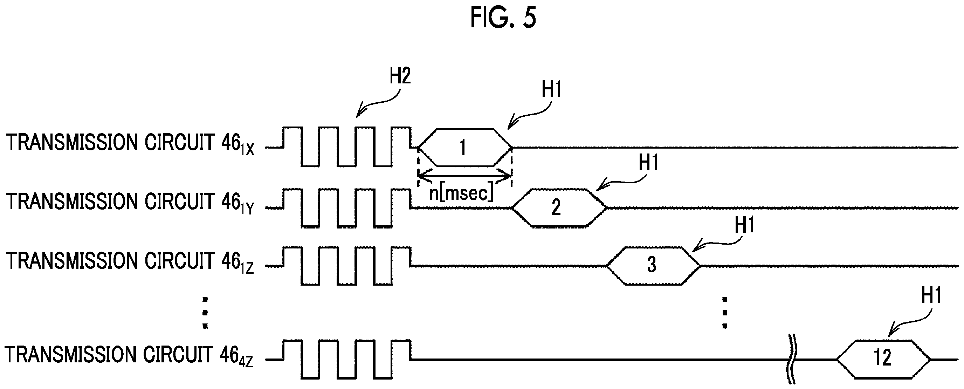

Specifically, as illustrated in Fig. 5 as an example, first, the output unit 64 outputs a second waveform H2 to all of the transmission circuits 46 at the same timing. Then, the output unit 64 outputs a first waveform H1 to all of the transmission circuits 46 at different timings after outputting the second waveform H2. In the embodiment, the output unit 64 outputs the first waveform H1 to all of the transmission circuits 46 according to a predetermined order. That is, after the magnetic field according to the second waveform H2 is generated from all of the transmission coils 49 at the same timing, the magnetic field according to the first waveform H1 is generated from each transmission coil 49 at different timings according to the predetermined order. The order of the transmission circuits 46 to which the output unit 64 outputs the first waveform H1 is not particularly limited. In the embodiment, as an example of identification information, numbers (for example, 1 to 12) are assigned to the respective transmission coils 49, and a case in which the output unit 64 outputs the first waveform H1 to the transmission circuit 46 corresponding to the transmission coil 49 in ascending order of the numbers will be described. In addition, information indicating the order of transmission circuits 46 to which the output unit 64 outputs the first waveform H1 is stored also in the overall controller 40.

In the embodiment, a case in which a period in during which one first waveform H1 is input is n milliseconds (for example, several tens of milliseconds) will be described. The numerical characters (1, 2, 3, ..., 12) of the first waveform H1 in Fig. 5 indicate the numbers assigned to the respective transmission coils 49.

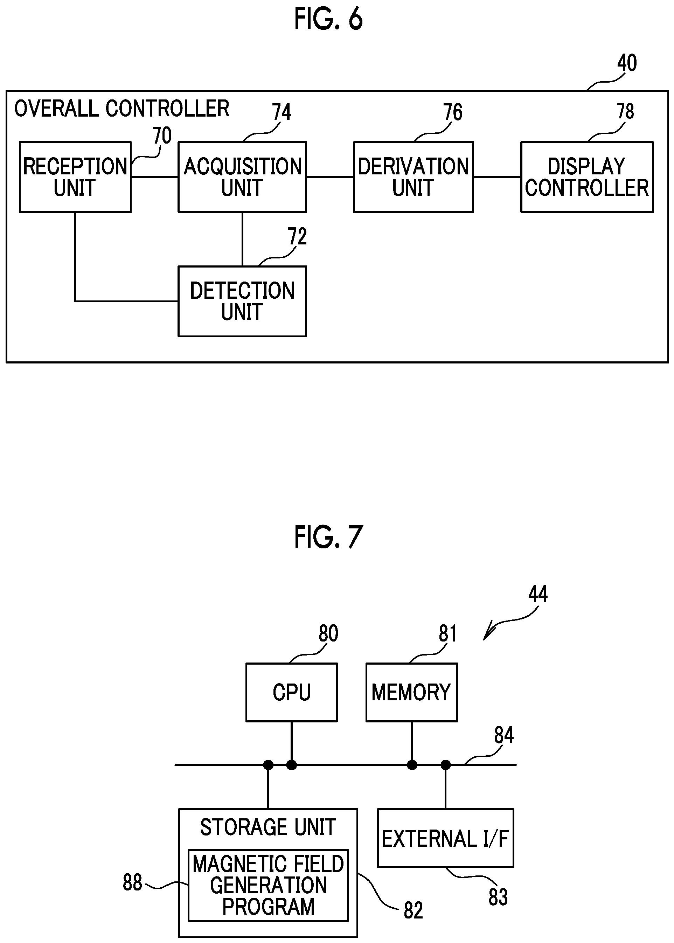

Next, the functional configuration of the overall controller 40 according to the embodiment will be described with reference to Fig. 6.

As illustrated in Fig. 6, the overall controller 40 includes a reception unit 70, a detection unit 72, an acquisition unit 74, a derivation unit 76, and a display controller 78.

As described above, the magnetic field generated by each transmission coil 49 is detected by each reception coil 23, and the detection signal according to the detected magnetic field is transmitted from the reception controller 20 of the endoscope 10 to the endoscope inspection device 12 via the I/F 29.

The reception unit 70 receives the detection signal transmitted from the reception controller 20 of the endoscope 10 via the I/F 53. The detection unit 72 detects the signal detected by the reception coil 23 according to the magnetic field generated by the transmission coil 49 by the second waveform H2, from the detection signal received by the reception unit 70. As a result, the detection unit 72 detects the timing at which the signal corresponding to the second waveform H2 is detected by the reception coil 23. Hereinafter, the timing at which the detection unit 72 detects the signal detected by the reception coil 23 according to the magnetic field generated by the transmission coil 49 by the second waveform H2 is referred to as the "synchronization timing".

The acquisition unit 74 acquires the signal corresponding to the first waveform H1, from the detection signal corresponding to each reception coil 23 received by the reception unit 70, on the basis of the synchronization timing detected by the detection unit 72 corresponding to each reception coil 23. Specifically, the acquisition unit 74 acquires the signal corresponding to the first waveform H1 every n milliseconds described above from the detection signal, with the synchronization timing as a reference. That is, the order in which the acquisition unit 74 acquires the first waveform H1 every n milliseconds corresponds to the order of the transmission coils 49 that have generated the magnetic field according to the first waveform H1. Accordingly, for each of the detection signals detected by the reception coils 23, the signal according to the first waveform H1 corresponding to each transmission coil 49 is acquired by the acquisition unit 74.

The derivation unit 76 derives the position and orientation of each reception coil 23 on the basis of the signal acquired by the acquisition unit 74. The method by which the derivation unit 76 derives the position and orientation of the reception coil 23 on the basis of the signal acquired by the acquisition unit 74 is not particularly limited, but for example, a technique disclosed in JP3432825B can be applied. In the technique disclosed in JP3432825B, an estimation value of a distance from a specific transmission coil 49 to a reception coil 23 is calculated from measurement values of the magnetic fields corresponding to the first waveform H1 generated by the transmission coils 49 and estimation values of orientations of the reception coils 23. Next, the estimation value of the position of the reception coil 23 is calculated from the estimation value of the distance from each transmission coil 49 to the reception coil 23, and the known position of the transmission coil 49. A new estimation value of the orientation of the reception coil 23 is calculated from the estimated position of the reception coil 23 and the measurement value of the magnetic field corresponding to the first waveform H1 of the reception coil 23. Then, the position and orientation of the reception coil 23 are derived by repeating the calculation of the estimation value of the distance from the transmission coil 49 to the reception coil 23 and the calculation of the estimation value of the position of the reception coil 23 by using the new estimation value of the orientation of the reception coil 23.

Further, the derivation unit 76 derives the shape of the insertion part 10A of the endoscope 10 on the basis of the derived position and orientation of each reception coil 23.

The display controller 78 performs control to display an endoscopic image that the image data input from the video processor 34 indicates, in a partial display region of the display unit 52. The display controller 78 performs control to display an image representing the insertion part 10A of the endoscope 10 according to the shape derived by the derivation unit 76, in a display region other than the display region where the endoscopic image is displayed, of the display unit 52. In a case where the endoscope inspection device 12 comprises a plurality of display units, the display controller 78 may perform control to display the endoscopic image and the image representing the insertion part 10A of the endoscope 10 on different display units.

Next, the hardware configuration of the transmission controller 44 according to the embodiment will be described with reference to Fig. 7.

As illustrated in Fig. 7, the transmission controller 44 includes a central processing unit (CPU) 80, a memory 81 as a temporary storage area, a non-volatile storage unit 82 such as a flash memory, and an external I/F 83 to which each transmission circuit 46 is connected. The CPU 80, the memory 81, the storage unit 82, and the external I/F 83 are connected to a bus 84.

In the storage unit 82, a magnetic field generation program 88 is stored. The CPU 80 reads the magnetic field generation program 88 from the storage unit 82 to expand the magnetic field generation program 88 in the memory 81, and executes the expanded magnetic field generation program 88. The CPU 80 executes the magnetic field generation program 88 to function as the first generation unit 60, the second generation unit 62, and the output unit 64 illustrated in Fig. 4. The reception controller 20 according to the embodiment is realized by the same hardware as the transmission controller 44.

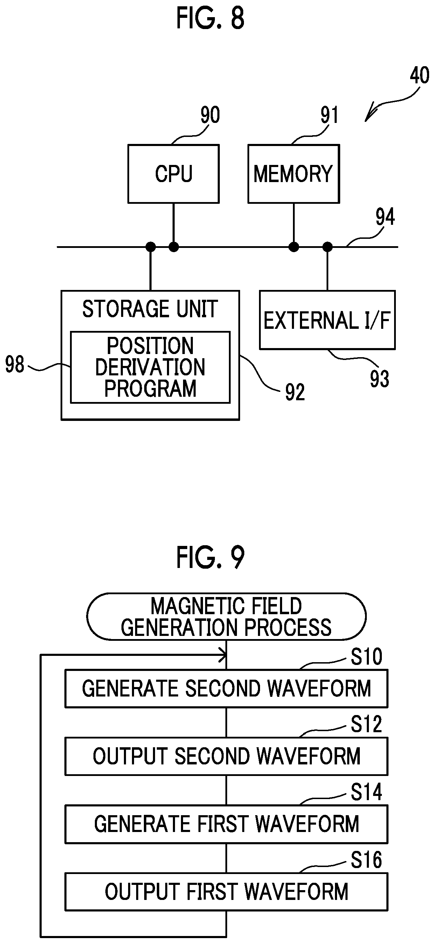

Next, the hardware configuration of the overall controller 40 according to the embodiment will be described with reference to Fig. 8.

As illustrated in Fig. 8, the overall controller 40 includes a CPU 90, a memory 91 as a temporary storage area, a non-volatile storage unit 92 such as a flash memory, and an external I/F 93 to which the video processor 34, the display unit 52, and the I/F 53 are connected. The CPU 90, the memory 91, the storage unit 92, and the external I/F 93 are connected to a bus 94.

In the storage unit 92, a position derivation program 98 is stored. The CPU 90 reads the position derivation program 98 from the storage unit 92 to expand the position derivation program 98 in the memory 91, and executes the expanded position derivation program 98. The CPU 90 executes the position derivation program 98 to function as the reception unit 70, the detection unit 72, the acquisition unit 74, the derivation unit 76, and the display controller 78 illustrated in Fig. 6.

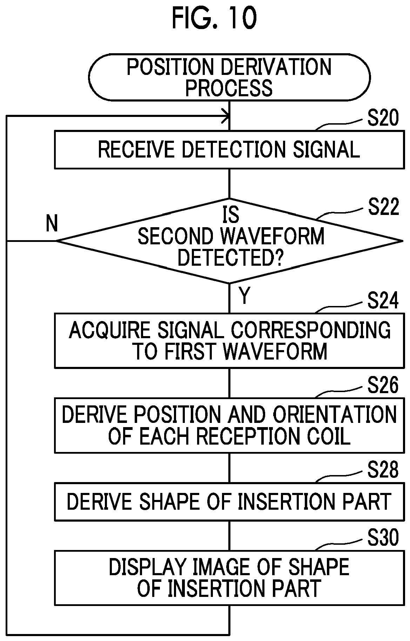

Next, the operation of the endoscope system 1 according to the embodiment will be described with reference to Figs. 9 and 10. The CPU 80 executes the magnetic field generation program 88, and thereby a magnetic field generation process illustrated in Fig. 9 is executed. The magnetic field generation process illustrated in Fig. 9 is executed in a case where the power switch of the transmission unit 41 is turned on, for example. In a case where the transmission unit 41 comprises a switch for switching on/off the magnetic field generation, the magnetic field generation process illustrated in Fig. 9 may be executed in a case where the switch is turned on.

The CPU 90 executes the position derivation program 98, and thereby a position derivation process illustrated in Fig. 10 is executed. The position derivation process illustrated in Fig. 10 is executed in a case where the power switch of the overall controller 40 is turned on, for example. In a case where the overall controller 40 comprises a switch for switching on/off the shape detection of the insertion part 10A, the position derivation process illustrated in Fig. 10 may be executed in a case where the switch is turned on.

In Step S10 of Fig. 9, the second generation unit 62 generates the second waveform H2. In Step S12, the output unit 64 outputs the second waveform H2 generated by the process of Step S10, to each of all of the transmission circuits 46.

In Step S14, the first generation unit 60 generates the first waveform H1. In Step S16, the output unit 64 outputs the first waveform H1 according to the predetermined order to each of all of the transmission circuits 46, as described above. In a case where the process of Step S16 is ended, the process returns to Step S10.

The magnetic field generation process illustrated in Fig. 9 is ended in a case where the power switch of the transmission unit 41 is turned off, for example. In a case where the transmission unit 41 comprises a switch for switching on/off the magnetic field generation, the magnetic field generation process illustrated in Fig. 9 may be ended in a case where the switch is turned off.

The magnetic field generated by each transmission coil 49 by the magnetic field generation process illustrated in Fig. 9 is detected by each reception coil 23, and the detection signal according to the detected magnetic field is transmitted from the reception controller 20 of the endoscope 10 to the endoscope inspection device 12 via the I/F 29.

In Step S20 of Fig. 10, the reception unit 70 receives the detection signal transmitted from the reception controller 20 of the endoscope 10 via the I/F 53. In Step S22, it is determined whether the detection unit 72 has detected the signal detected by the reception coil 23 according to the magnetic field generated by the transmission coil 49 by the second waveform H2, from the detection signal received in the process of Step S20. In a case where the determination is negative, the process returns to Step S20, and in a case where the determination is affirmative, the processing proceeds to Step S24.

In Step S24, the acquisition unit 74 acquires the signal corresponding to the first waveform H1 every n milliseconds from each detection signal received in the process of Step S20, with the synchronization timing detected in the process of Step S22 as a reference. In Step S26, the derivation unit 76 derives the position and orientation of each reception coil 23 on the basis of the signal acquired in the process of Step S24, as described above.

In Step S28, the derivation unit 76 derives the shape of the insertion part 10A of the endoscope 10 on the basis of the position and orientation of each reception coil 23 derived in the process of Step S26. In Step S30, the display controller 78 performs control to display the image representing the insertion part 10A of the endoscope 10 according to the shape derived in the process of Step S28, in a display region other than the display region where the endoscopic image is displayed, of the display unit 52. In a case where the process of Step S30 is ended, the process returns to Step S20.

The position derivation process illustrated in Fig. 10 is ended in a case where the power switch of the overall controller 40 is turned off, for example. In a case where the overall controller 40 comprises a switch for switching on/off the shape detection of the insertion part 10A, the position derivation process illustrated in Fig. 10 may be ended in a case where the switch is turned off.

As described above, according to the embodiment, after the transmission coils 49 are driven with the second waveform H2, the transmission coils 49 are driven with the first waveform H1. Further, the signal corresponding to the first waveform H1 detected by each reception coil 23 is acquired on the basis of the timing at which the signal corresponding to the second waveform H2 is detected by the reception coil 23. Then, the position of each reception coil 23 is derived on the basis of the acquired signal. Accordingly, the transmission unit 41 and the reception unit 21 can be wirelessly connected without providing a wireless communication unit for notifying a timing at which the magnetic field is generated. That is, according to the embodiment, the transmission controller 44 stores, in the storage unit 92 of the overall controller 40 in advance, information on the drive timing to drive each transmission coil 49 with the first waveform after driving each transmission coil 49 with the second waveform H2, and the derivation unit derives the position of each of the plurality of magnetic field detecting elements on the basis of the information on the drive timing stored in the overall controller 40. As a result, the transmission unit 41 including the transmission controller 44, the transmission circuits 46 (drive unit), and the transmission coils 49, and the reception coils 23, the derivation unit 76, the acquisition unit 74, and the storage unit 92 which are on the reception side can be wirelessly connected (wireless connection refers to that the transmission unit 41 including the transmission controller 44, the transmission circuits 46, and the transmission coils 49, and the reception coils 23, the derivation unit 76, the acquisition unit 74, and the storage unit 92 which are on the reception side are not electrically connected). The information on the drive timing is information that specifies a timing from driving each transmission coil 49 with the second waveform to driving each transmission coil 49 with the first waveform. Examples of the information on the drive timing include the time from driving each transmission coil 49 with the second waveform to driving each transmission coil 49 with the first waveform, the order in which each transmission coil 49 is driven with the first waveform, and the like.

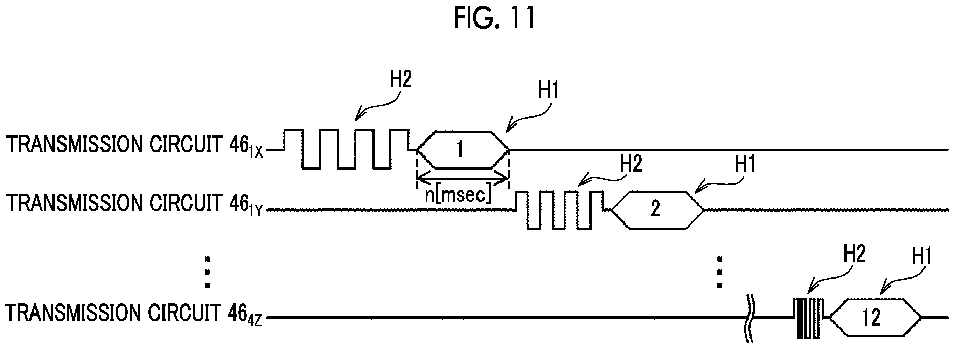

In the first embodiment, the output unit 64 may output the second waveform H2 to each of all of the transmission circuits 46 at different timings. In this case, as illustrated in Fig. 11 as an example, a form is exemplified in which the first waveform H1 is output to each of all of the transmission circuits 46 immediately after the second waveform H2 is output, according to the predetermined order. In this form example, the combination of the second waveform H2 and the first waveform H1 is input to each transmission circuit 46 at different timings. Further, in this form example, the second waveforms H2 corresponding to the transmission circuits 46 can be distinguished from each other by having different frequencies. The second waveforms H2 corresponding to the transmission circuits 46 may be distinguishable from each other by differentiating at least one of the frequencies, phases, amplitudes, or waveform shapes.

In this form example, the acquisition unit 74 acquires the signal corresponding to the first waveform H1 every n milliseconds from each detection signal, with the synchronization timing as a reference. Further, the acquisition unit 74 can specify which transmission coil 49 the acquired signal corresponds to, according to the frequency of the signal detected by the detection unit 72.

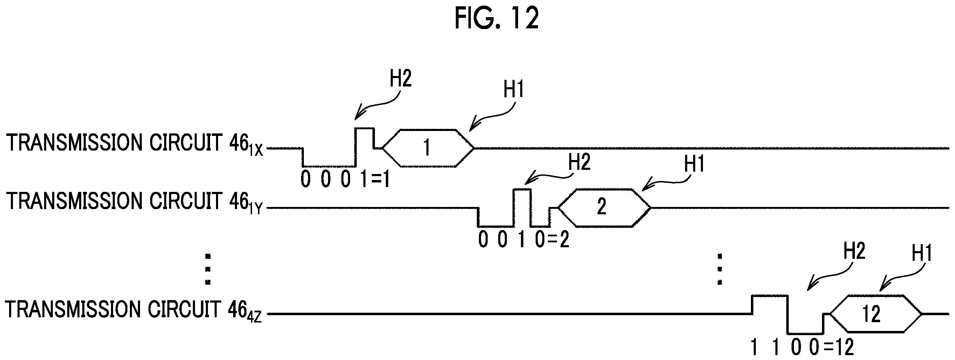

In this form example, as illustrated in Fig. 12 as an example, a waveform representing the number as an example of the identification information of each transmission coil 49 may be applied as the second waveform H2.

### [Second Embodiment]

A second embodiment of the technique of the present disclosure will be described. Since the configuration (refer to Figs. 1 to 3) of the endoscope system 1 is the same as that in the first embodiment, the description thereof will be omitted.

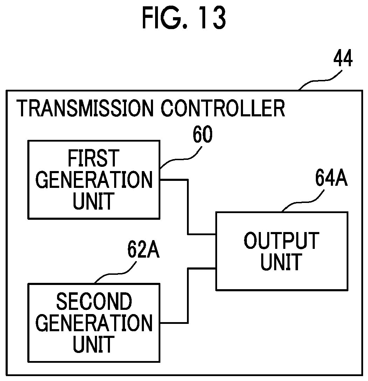

The functional configuration of the transmission controller 44 according to the embodiment will be described with reference to Fig. 13. In Fig. 13, the functional unit having the same function as that in Fig. 4 is denoted by the same reference numeral, and the description thereof will be omitted. As illustrated in Fig. 13, the transmission controller 44 includes the first generation unit 60, a second generation unit 62A, and an output unit 64A.

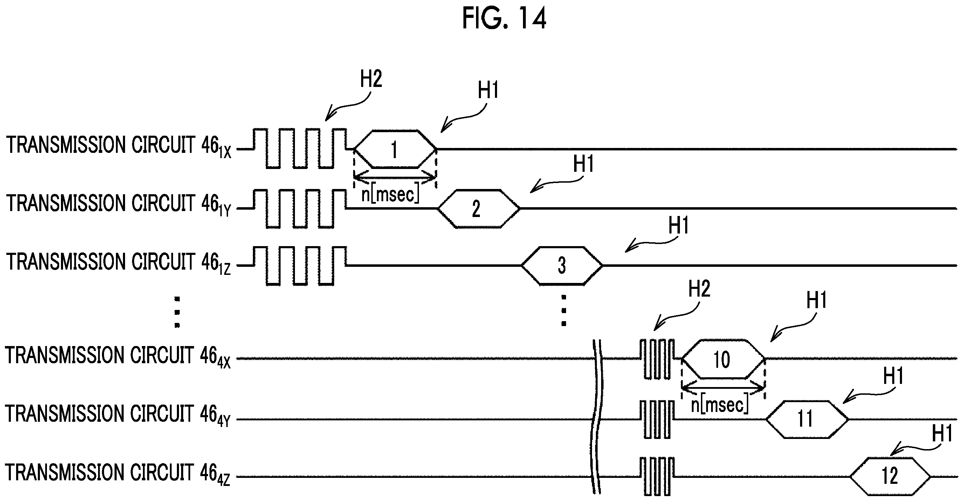

As illustrated in Fig. 14 as an example, the second generation unit 62A generates the same second waveform H2 for three transmission coils 49 in each combination. Further, the second generation unit 62A generates the second waveforms H2 which can be distinguished by differentiating at least one of the frequencies, phases, amplitudes, or waveform shapes, for the combinations each having three transmission coils 49. In the embodiment, the second waveforms H2 can be distinguished from each other by differentiating the frequencies for the combinations each having three transmission coils 49. Further, also in the embodiment, the first waveform H1 and the second waveform H2 are different in at least one of the frequencies, phases, amplitudes, or waveform shapes, so that the first waveform H1 and the second waveform H2 can be distinguished from each other.

The output unit 64A outputs the second waveform H2 to the transmission circuits 46, and then outputs the first waveform H1 to the transmission circuits 46. Specifically, as illustrated in Fig. 14, the output unit 64A outputs the second waveform H2 to each of the transmission circuits 46 corresponding to three transmission coils 49 in each combination, at the same timing. The output unit 64A outputs the first waveform H1 to each of the transmission circuits 46 corresponding to three transmission coils 49 in each combination, at different timings after outputting the second waveform H2. In the embodiment, similar to the first embodiment, the output unit 64A outputs the first waveform H1 to each of the transmission circuits 46 corresponding to three transmission coils 49 in each combination, in the predetermined order.

The output unit 64A performs outputting of the first waveform H1 and the second waveform H2 at different timings between the combinations. In the embodiment, the output unit 64A performs outputting of the first waveform H1 and the second waveform H2 in order from the combination having the smallest number of the transmission coils 49.

That is, after the magnetic field according to the second waveform H2 is generated from three transmission coils 49 in each combination at the same timing, the magnetic field according to the first waveform H1 is generated from three transmission coils 49 in each combination according to the predetermined order. The magnetic fields according to the first waveform H1 and the second waveform H2 are generated from three transmission coils 49 at different timings between the combinations.

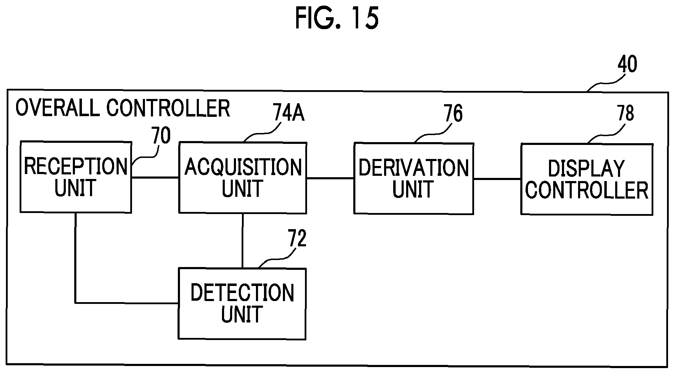

Next, the functional configuration of the overall controller 40 according to the embodiment will be described with reference to Fig. 15. In Fig. 15, the functional units having the same functions as those in Fig. 6 are denoted by the same reference numerals, and the description thereof will be omitted. As illustrated in Fig. 15, the overall controller 40 includes the reception unit 70, the detection unit 72, an acquisition unit 74A, the derivation unit 76, and the display controller 78.

The acquisition unit 74A acquires the signal corresponding to the first waveform H1, from the detection signal corresponding to each reception coil 23 received by the reception unit 70, on the basis of the synchronization timing detected by the detection unit 72 corresponding to each reception coil 23. Specifically, the acquisition unit 74A acquires the signal corresponding to the first waveform H1 three times every n milliseconds from the detection signal, with the synchronization timing as a reference. The acquisition unit 74A specifies by which combination of three transmission coils 49 the acquired signal is due to the magnetic field according to the first waveform H1 generated, from the frequency of the signal detected by the detection unit 72. That is, the acquisition unit 74A can associate the acquired signal with the transmission coil 49 from the frequency of the signal detected by the detection unit 72 and the order in which the first waveform H1 is acquired every n milliseconds. The acquisition unit 74A performs this process for each of four combinations each having three transmission coils. As a result, for each reception coil 23, the signal corresponding to the magnetic field generated according to the first waveform H1 by each transmission coil 49 is acquired.

Since the hardware configuration of the transmission controller 44 and the hardware configuration of the overall controller 40 are the same as those in the first embodiment, the description thereof will be omitted.

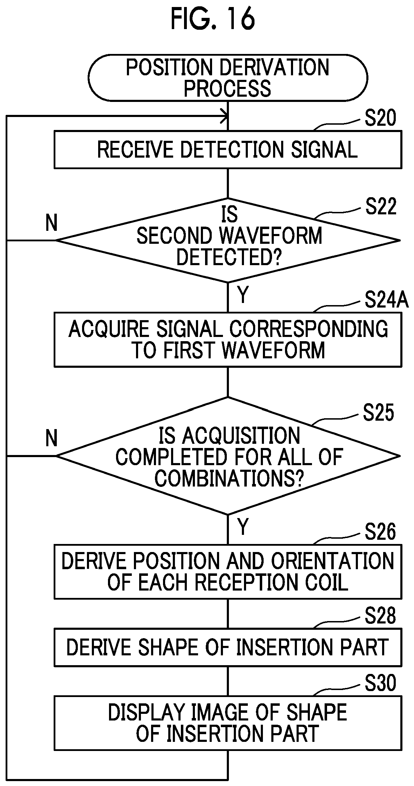

Next, the operation of the endoscope system 1 according to the embodiment will be described with reference to Fig. 16. Since the magnetic field generation process according to the embodiment is the same as the magnetic field generation process (refer to Fig. 9) according to the first embodiment except that the magnetic field generation process according to the embodiment is repeatedly executed for each combination of three transmission circuits 46, the description thereof will be omitted. In Fig. 16, steps executing the same process as those in Fig. 10 are denoted by the same step numbers as those in Fig. 10, and the description thereof will be omitted.

In a case where the determination of Step S22 of Fig. 16 is affirmative, the process proceeds to Step S24A. In Step S24A, the acquisition unit 74A acquires the signal corresponding to the first waveform H1 three times every n milliseconds from each detection signal received in the process of Step S20, with the synchronization timing detected in the process of Step S22 as a reference, as described above. The acquisition unit 74A specifies by which combination of three transmission coils 49 the acquired signal is due to the magnetic field according to the first waveform H1 generated, from the frequency of the signal detected in the process of Step S22.

In Step S25, the acquisition unit 74A determines whether the process of Step S24A is completed for all of four combinations each having three transmission coils 49. In a case where the determination is affirmative, the process proceeds to Step S26, and in a case where the determination is negative, the process returns to Step S20.

As described above, according to the embodiment, the magnetic fields are generated by the first waveform H1 and the second waveform H2 for each combination of three transmission coils 49. Accordingly, the same effects as those in the first embodiment can be obtained. Further, since the number of transmission coils 49 driven with the second waveform H2 at the same time is three, the number can be reduced to three by sharing a circuit that generates an analog signal representing the second waveform H2. Since three transmission coils 49 that are orthogonal to each other on three axes are driven as one set with the second waveform H2 at the same timing, signals with an intensity corresponding to the distance between the transmission coils 49 and the reception coils 23 can be obtained regardless of the orientation of each reception coil 23. In the embodiment, the same second waveform H2 is generated for each combination of three transmission coils 49, but the present disclosure is not limited thereto, and the same second waveform H2 may be generated for each combination of two or more transmission coils with different orientations of magnetic field generation. Further, adjacent coils may be grouped, or non-adjacent coils may be grouped. By generating the same second waveform H2 for each combination of two or more transmission coils with different orientations of magnetic field generation, the magnetic field can be detected by the reception coil regardless of the orientation of the magnetic field generated by the transmission coil.

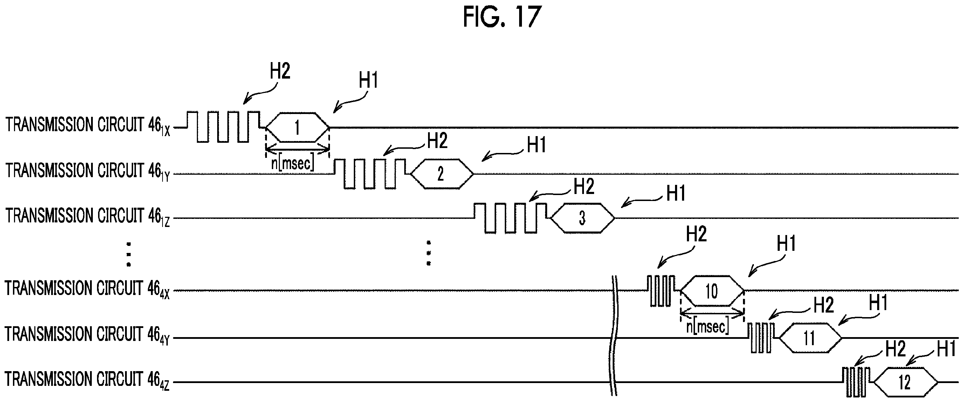

In the second embodiment, the output unit 64Amay output the same second waveform H2 to each of the transmission circuits 46 in each combination, at different timings. In this case, as illustrated in Fig. 17 as an example, a form is exemplified in which the first waveform H1 is output to each of the transmission circuits 46 in each combination immediately after the second waveform H2 is output, according to the predetermined order.

In each embodiment described above, the transmission coil 49 to be driven with the second waveform H2 may be selected from among all of the transmission coils 49 according to the previously derived position of each reception coil 23. In this case, in the first embodiment, a form is exemplified in which a predetermined number (for example, four) of transmission coils 49 are selected in order of proximity to the previously derived position of each reception coil 23 (for example, in ascending order of the average value or the total value of the distances to the reception coils 23). In this case, in the second embodiment, a form is exemplified in which a predetermined number (for example, one) of transmission coils 49 are selected from three transmission coils 49 in each combination in order of proximity to the reception coil 23.

In this case, for example, the CPU 80 functions as a selection unit that selects the transmission coil 49 to be driven with the second waveform H2. As a result, it is possible to reduce the number of components for detecting the synchronization timing.

In this form example, the transmission coil 49 to be driven with the second waveform H2 may be selected from all of the transmission coils 49 according to the intensity of the signal corresponding to the first waveform H1 detected by each reception coil 23, instead of the position of each reception coil 23. In this case, in the first embodiment, a form is exemplified in which a predetermined number (for example, four) of transmission coils 49 are selected in descending order of the intensity of the signal corresponding to the first waveform H1 previously detected by each reception coil 23. In this case, in the second embodiment, a form is exemplified in which a predetermined number (for example, one) of transmission coils 49 are selected from three transmission coils 49 in each combination, in descending order of the intensity of the signal corresponding to the first waveform H1 detected by each reception coil 23. In this case, for example, the CPU 80 functions as a selection unit that selects the transmission coil 49 to be driven with the second waveform H2. As a result, it is possible to reduce the number of components for detecting the synchronization timing.

In each embodiment described above, the reception coil 23 as the detection target of the signal corresponding to the second waveform H2 may be selected from among all of the reception coils 23 according to the previously derived position of each reception coil 23. In this case, a form is exemplified in which the reception coil 23 of which the previously derived position is closest to each transmission coil 49 is selected. In this case, for example, the CPU 90 or a CPU of the reception controller 20 functions as a selection unit that selects the reception coil 23 as the detection target of the signal corresponding to the second waveform H2. As a result, it is possible to reduce the number of components for detecting the synchronization timing.

In this form example, the reception coil 23 as the detection target of the signal corresponding to the second waveform H2 may be selected from all of the reception coils 23 according to the intensity of the signal corresponding to the first waveform H1 detected by each reception coil 23, instead of the position of each reception coil 23. In this case, a form is exemplified in which the reception coil 23 with the highest intensity of the signal corresponding to the previously detected first waveform H1 is selected. In this case, for example, the CPU 90 or a CPU of the reception controller 20 functions as a selection unit that selects the reception coil 23 as the detection target of the signal corresponding to the second waveform H2. As a result, it is possible to reduce the number of components for detecting the synchronization timing.

In each embodiment described above, the reception controller 20 may include at least a part of the functions of the overall controller 40. In this case, a form is exemplified in which the reception controller 20 comprises the detection unit 72, and the acquisition units 74 and 74A.

In each embodiment described above, a case in which coils are applied as the magnetic field generating elements and the magnetic field detecting elements has been described, but the present disclosure is not limited thereto. As the magnetic field generating elements, for example, elements that generate magnetic fields, such as spin torque oscillation elements may be applied. Further, as the magnetic field detecting elements, for example, elements that detect magnetic fields, such as Hall elements, and magneto resistive (MR) elements may be applied.

In each embodiment described above, for example, the following various processors can be used as the hardware structure of processing units executing various processes such as the first generation unit 60, the second generation unit 62 and 62A, the output units 64 and 64A, the reception unit 70, the detection unit 72, the acquisition units 74 and 74A, the derivation unit 76, and the display controller 78. The various processors include, for example, a programmable logic device (PLD) that is a processor of which the circuit configuration can be changed after manufacture, such as a field-programmable gate array (FPGA), and a dedicated electric circuit that is a processor having a dedicated circuit configuration designed to execute a specific process, such as an application specific integrated circuit (ASIC), in addition to the CPU that is a general-purpose processor which executes software (program) to function as various processing units as described above.

One processing unit may be configured by one of the various processors or a combination of the same or different kinds of two or more processors (for example, a combination of a plurality of FPGAs or a combination of a CPU and an FPGA). In addition, a plurality of processing units may be configured by one processor.

As an example where a plurality of processing units are configured by one processor, first, there is a form where one processor is configured by a combination of one or more CPUs and software as typified by a computer, such as a client and a server, and this processor functions as a plurality of processing units. Second, there is a form where a processor fulfilling the functions of the entire system including a plurality of processing units by one integrated circuit (IC) chip as typified by a system on chip (SoC) or the like is used. In this manner, various processing units are configured by using one or more of the above-described various processors as hardware structures.

Further, specifically, an electric circuit (circuitry) obtained by combining circuit elements, such as semiconductor elements, can be used as the hardware structure of the various processors.

In each embodiment described above, an aspect in which the magnetic field generation program 88 is stored (installed) in the storage unit 82 in advance is described, but the present disclosure is not limited thereto. The magnetic field generation program 88 may be provided by being recorded in a recording medium such as a compact disk read only memory (CD-ROM), a digital versatile disk read only memory (DVD-ROM), and a Universal Serial Bus (USB) memory. In addition, the magnetic field generation program 88 may be downloaded from an external device through a network.

In each embodiment described above, an aspect in which the position derivation program 98 is stored (installed) in the storage unit 92 in advance is described, but the present disclosure is not limited thereto. The position derivation program 98 may be provided by being recorded in a recording medium such as a CD-ROM, a DVD-ROM, and a USB memory. In addition, the position derivation program 98 may be downloaded from an external device through a network.

### Explanation of References

1: endoscope system
10: endoscope
10A: insertion part
10B: operation part
11: cable
12: endoscope inspection device
20: reception controller
21, 70: reception unit
22: reception coil unit
23₁ to 23₁₆: reception coil
24₁ to 24₁₆: reception circuit
26₁ to 26₁₆: ADC29, 53: I/F30: image sensor
34: video processor
36: light source
40: overall controller
41: transmission unit
42: transmission control unit
44: transmission controller
46_{1X}, 46_{1Y}, 46_{1Z} to 46_{4X}, 46_{4Y}, 46_{4Z}: transmission circuit
48: transmission coil unit
49_{1X}, 49_{1Y}, 49_{1Z} to 49_{4X}, 49_{4Y}, 49_{4Z}: transmission coil
52: display unit
60: first generation unit
62, 62A: second generation unit
64, 64A: output unit
72: detection unit
74, 74A: acquisition unit
76: derivation unit
78: display controller
80, 90: CPU81, 91: memory
82, 92: storage unit
83, 93: external I/F84, 94: bus
88: magnetic field generation program
98: position derivation program
H1: first waveform
H2: second waveform
W: subject

## Claims

1. An endoscope system comprising:
• a plurality of magnetic field generating elements (49);
• a plurality of magnetic field detecting elements (23);
• a first generation unit (60) configured to generate a first waveform for detecting a position of an insertion part of an endoscope to be inserted into a subject;
• a second generation unit (62, 62A) configured to generate a second waveform, which is different from the first waveform in at least one of a frequency, a phase, an amplitude, or a waveform shape and is distinguishable from the first waveform;
**characterized in that** the endoscope system further comprises:
• a drive unit configured to drive the plurality of magnetic field generating elements (49) with the second waveform and further configured to drive the plurality of magnetic field generating elements with the first waveform after driving the plurality of magnetic field generating elements (49) with the second waveform;
• a detection unit (72) configured to detect, for each of the plurality of magnetic field detecting elements, a synchronization timing corresponding to the detection of the second waveform by each of the plurality of magnetic field detecting elements;
• an acquisition unit (74, 74A) configured to acquire a signal corresponding to the first waveform detected by each of the plurality of magnetic field detecting elements on the basis of the synchronization timing detected for each of the plurality of magnetic field detecting elements; and
• a derivation unit configured to derive a position of each of the plurality of magnetic field detecting elements (23) on the basis of the signal acquired by the acquisition unit (74, 74A).

2. The endoscope system according to claim 1,wherein the drive unit is further configured to simultaneously drive each of the plurality of magnetic field generating elements (49) with the second waveform.

3. The endoscope system according to claim 1,wherein the plurality of magnetic field generating elements (49) are grouped together into combinations of three magnetic field generating elements each, and the drive unit is further configured to simultaneously drive the three magnetic field generating elements in each combination with the same second waveform the second waveform of a combination being distinguishable in at least one of frequency, phase, amplitude or waveform shape from the second waveform of another combination, the combinations being driven at a different timing.

4. The endoscope system according to claim 1,wherein the plurality of magnetic field generating elements (49) are grouped together into combinations of three magnetic field generating elements each, and the drive unit is further configured to drive the three magnetic field generating elements in each combination with the same second waveform at a different timing, the second waveform of a combination being distinguishable in at least one of frequency, phase, amplitude or waveform shape from the second waveform of another combination, the combinations being driven at a different timing.

5. The endoscope system according to claim 1,wherein the plurality of magnetic field generating elements (49) are grouped together into combinations of two or more magnetic field generating elements with different orientations of magnetic field generation, and the drive unit is further configured to simultaneously drive the magnetic field generating elements in each combination with the same second waveform, the second waveform of a combination being distinguishable in at least one of frequency, phase, amplitude or waveform shape from the second waveform of another combination, the combinations being driven at a different timing.

6. The endoscope system according to claim 1,wherein the plurality of magnetic field generating elements (49) are grouped together into combinations of two or more magnetic field generating elements with different orientations of magnetic field generation, and the drive unit is further configured to drive the magnetic field generating elements in each combination with the same second waveform at a different timing, the second waveform of a combination being distinguishable in at least one of frequency, phase, amplitude or waveform shape from the second waveform of another combination, the combinations being driven at a different timing.

7. The endoscope system according to claim 1,wherein the drive unit is further configured to drive each of the plurality of magnetic field generating elements (49) with a second waveform that is different in at least one of a frequency, a phase, an amplitude, or a waveform shape and distinguishable from another second waveform, each of the plurality of magnetic field generating elements (49) being driven at a different timing.

8. The endoscope system according to any one of claims 2 to 7,wherein the second waveforms are distinguishable from each other by having different frequencies or being waveforms representing identification information of the magnetic field generating element (49) to be driven.

9. The endoscope system according to any one of claims 1 to 8,wherein the acquisition unit is further configured to acquire the signal corresponding to the first waveform detected by each of the plurality of magnetic field detecting elements (23) on the basis of the synchronization timing at which the signal corresponding to the second waveform is detected by the plurality of magnetic field detecting elements (23).

10. The endoscope system according to any one of claims 1 to 9, further comprising: a selection unit configured to select the magnetic field generating element (49) to be driven with the second waveform, according to the position of each of the plurality of magnetic field detecting elements (23) previously derived by the derivation unit (76) or according to an intensity of the signal corresponding to the first waveform previously detected by the plurality of magnetic field detecting elements, wherein the drive unit is further configured to drive the magnetic field generating element selected by the selection unit, with the second waveform.

11. The endoscope system according to any one of claims 1 to 9, further comprising: a selection unit configured to select the magnetic field detecting element (23) as a detection target of the signal corresponding to the second waveform, according to the position of each of the plurality of magnetic field detecting elements (23) previously derived by the derivation unit (76) or according to an intensity of the signal corresponding to the first waveform previously detected by the plurality of magnetic field detecting elements, wherein the acquisition unit is further configured to acquire the signal corresponding to the first waveform from each of the plurality of magnetic field detecting elements on the basis of the synchronization timing at which the signal corresponding to the second waveform is detected by the magnetic field detecting element selected by the selection unit.

12. The endoscope system according to any one of claims 1 to 11, further comprising: a storage unit configured to store in advance information on drive timing to drive the magnetic field generating element with the first waveform after driving the magnetic field generating element with the second waveform, wherein the derivation unit is further configured to derive a position of each of the plurality of magnetic field detecting elements on the basis of the information on the drive timing stored in the storage unit.

13. The endoscope system according to any one of claims 1 to 12,wherein the magnetic field generating elements (49), the first generation unit, the second generation unit, and the drive unit are not electrically connected to the magnetic field detecting elements (23), the acquisition unit (74, 74A), and the derivation unit (76).

14. The endoscope system according to claim 12,wherein the magnetic field generating elements (49), the first generation unit (60), the second generation unit (62, 62A), and the drive unit are not electrically connected to the magnetic field detecting elements, the acquisition unit, the derivation unit, and the storage unit.

15. A position derivation method by an endoscope system including a plurality of magnetic field generating elements (49) and a plurality of magnetic field detecting elements (23), the position derivation method comprising the consecutive steps of:
• generating a first waveform for detecting a position of an insertion part of an endoscope to be inserted into a subject;
• generating a second waveform for detecting a synchronization timing, which is different from the first waveform in at least one of a frequency, a phase, an amplitude, or a waveform shape and is distinguishable from the first waveform;
• driving the plurality of magnetic field generating elements (49) with the second waveform;
• detecting, for each of the plurality of magnetic field generating elements (49), a synchronization timing corresponding to the detection of the second waveform by each of the plurality of magnetic field generating elements (49);
• driving the plurality of magnetic field generating elements (49) with the first waveform after driving the plurality of magnetic field generating elements (49) with the second waveform;
• acquiring a signal corresponding to the first waveform detected by each of the plurality of magnetic field detecting elements (23) on the basis of the synchronization timing detected for each of the plurality of magnetic field detecting elements (23); and
• deriving a position of each of the plurality of magnetic field detecting elements (23) on the basis of the acquired signal.

## Patentansprüche

1. Ein Endoskopsystem, aufweisend:
• eine Vielzahl von Magnetfelderzeugungselementen (49);
• eine Vielzahl von Magnetfelderfassungselementen (23);
• eine erste Erzeugungseinheit (60), die so konfiguriert ist, dass sie eine erste Wellenform zum Erfassen einer Position eines Einführungsteils eines Endoskops, das in ein Subjekt eingeführt werden soll, erzeugt;
• eine zweite Erzeugungseinheit (62, 62A), die so konfiguriert ist, dass sie eine zweite Wellenform erzeugt, die sich von der ersten Wellenform in mindestens einer Frequenz, einer Phase, einer Amplitude oder einer Wellenformform unterscheidet und von der ersten Wellenform unterscheidbar ist;
**dadurch gekennzeichnet, dass** das Endoskopsystem ferner umfasst:
• eine Antriebs- bzw. Ansteuerungseinheit, die so konfiguriert ist, dass sie die Vielzahl von Magnetfelderzeugungselementen (49) mit der zweiten Wellenform ansteuert, und ferner so konfiguriert ist, dass sie die Vielzahl von Magnetfelderzeugungselementen mit der ersten Wellenform ansteuert, nachdem sie die Vielzahl von Magnetfelderzeugungselementen (49) mit der zweiten Wellenform angesteuert hat;
• eine Erfassungseinheit (72), die so konfiguriert ist, dass sie für jedes der mehreren Magnetfelderfassungselemente eine Synchronisationszeit erfasst, die der Erfassung der zweiten Wellenform durch jedes der mehreren Magnetfelderfassungselemente entspricht;
• eine Erfassungseinheit (74, 74A), die so konfiguriert ist, dass sie ein Signal erfasst, das der ersten Wellenform entspricht, die von jedem der mehreren Magnetfelderfassungselemente auf der Grundlage der Synchronisationszeit erfasst wird, die für jedes der mehreren Magnetfelderfassungselemente erfasst wird; und
• eine Ableitungseinheit, die so konfiguriert ist, dass sie eine Position jedes der mehreren Magnetfelderfassungselemente (23) auf der Grundlage des von der Erfassungseinheit (74, 74A) erfassten Signals ableitet.

2. Endoskopsystem nach Anspruch 1, wobei die Antriebseinheit ferner so konfiguriert ist, dass sie jedes der mehreren Magnetfelderzeugungselemente (49) gleichzeitig mit der zweiten Wellenform ansteuert.

3. Endoskopsystem nach Anspruch 1, wobei die mehreren Magnetfelderzeugungselemente (49) zu Kombinationen von jeweils drei Magnetfelderzeugungselementen gruppiert sind und die Ansteuerungseinheit ferner so konfiguriert ist, dass sie die drei Magnetfelderzeugungselemente in jeder Kombination gleichzeitig mit derselben zweiten Wellenform ansteuert, wobei sich die zweite Wellenform einer Kombination in mindestens einer der folgenden Eigenschaften von der zweiten Wellenform einer anderen Kombination unterscheidet: Frequenz, Phase, Amplitude oder Wellenformform, wobei die Kombinationen zu einem unterschiedlichen Zeitpunkt angesteuert werden.

4. Endoskopsystem nach Anspruch 1, wobei die mehreren Magnetfelderzeugungselemente (49) zu Kombinationen von jeweils drei Magnetfelderzeugungselementen gruppiert sind und die Ansteuerungseinheit ferner so konfiguriert ist, dass sie die drei Magnetfelderzeugungselemente in jeder Kombination mit der gleichen zweiten Wellenform zu einem unterschiedlichen Zeitpunkt ansteuert, wobei die zweite Wellenform einer Kombination in mindestens einer der Eigenschaften Frequenz, Phase, Amplitude oder Wellenformform von der zweiten Wellenform einer anderen Kombination unterscheidbar ist, wobei die Kombinationen zu einem unterschiedlichen Zeitpunkt angesteuert werden.

5. Endoskopsystem nach Anspruch 1, wobei die mehreren Magnetfelderzeugungselemente (49) zu Kombinationen von zwei oder mehr Magnetfelderzeugungselementen mit unterschiedlichen Ausrichtungen der Magnetfelderzeugung gruppiert sind und die Antriebseinheit ferner so konfiguriert ist, dass sie die Magnetfelderzeugungselemente in jeder Kombination gleichzeitig mit derselben zweiten Wellenform ansteuert, wobei sich die zweite Wellenform einer Kombination in mindestens einer der folgenden Eigenschaften von der zweiten Wellenform einer anderen Kombination unterscheidet: Frequenz, Phase, Amplitude oder Wellenformform, wobei die Kombinationen zu einem unterschiedlichen Zeitpunkt angesteuert werden.

6. Endoskopsystem nach Anspruch 1, wobei die mehreren Magnetfelderzeugungselemente (49) zu Kombinationen von zwei oder mehr Magnetfelderzeugungselementen mit unterschiedlichen Ausrichtungen der Magnetfelderzeugung gruppiert sind und die Antriebseinheit ferner so konfiguriert ist, dass sie die Magnetfelderzeugungselemente in jeder Kombination mit der gleichen zweiten Wellenform zu einem unterschiedlichen Zeitpunkt ansteuert, wobei die zweite Wellenform einer Kombination in mindestens einer der Eigenschaften Frequenz, Phase, Amplitude oder Wellenformform von der zweiten Wellenform einer anderen Kombination unterscheidbar ist, wobei die Kombinationen zu einem unterschiedlichen Zeitpunkt angesteuert werden.

7. Endoskopsystem nach Anspruch 1, wobei die Antriebseinheit ferner so konfiguriert ist, dass sie jedes der mehreren Magnetfelderzeugungselemente (49) mit einer zweiten Wellenform ansteuert, die sich in mindestens einer Frequenz, einer Phase, einer Amplitude oder einer Wellenformform unterscheidet und von einer anderen zweiten Wellenform unterscheidbar ist, wobei jedes der mehreren Magnetfelderzeugungselemente (49) zu einem unterschiedlichen Zeitpunkt angesteuert wird.

8. Endoskopsystem nach einem der Ansprüche 2 bis 7, wobei die zweiten Wellenformen voneinander unterscheidbar sind, indem sie unterschiedliche Frequenzen haben oder Wellenformen sind, die Identifikationsinformationen des anzusteuernden Magnetfelderzeugungselements (49) darstellen.

9. Endoskopsystem nach einem der Ansprüche 1 bis 8, wobei die Erfassungseinheit ferner so konfiguriert ist, dass sie das Signal, das der ersten Wellenform entspricht, die von jedem der mehreren Magnetfelderfassungselemente (23) erfasst wird, auf der Grundlage des Synchronisationszeitpunkts erfasst, zu dem das der zweiten Wellenform entsprechende Signal von den mehreren Magnetfelderfassungselementen (23) erfasst wird.

10. Endoskopsystem nach einem der Ansprüche 1 bis 9, das ferner umfasst: eine Auswahleinheit, die so konfiguriert ist, dass sie das Magnetfelderzeugungselement (49), das mit der zweiten Wellenform angesteuert werden soll, entsprechend der Position jedes der mehreren Magnetfelderfassungselemente (23), die zuvor von der Ableitungseinheit (76) abgeleitet wurden, oder entsprechend einer Intensität des Signals auswählt, das der ersten Wellenform entspricht, die zuvor von den mehreren Magnetfelderfassungselementen erfasst wurde, wobei die Ansteuerungseinheit ferner so konfiguriert ist, dass sie das von der Auswahleinheit ausgewählte Magnetfelderzeugungselement mit der zweiten Wellenform ansteuert.

11. Endoskopsystem nach einem der Ansprüche 1 bis 9, ferner umfassend: eine Auswahleinheit, die so konfiguriert ist, dass sie das Magnetfelderfassungselement (23) als ein Erfassungsziel des der zweiten Wellenform entsprechenden Signals auswählt, und zwar gemäß der Position jedes der mehreren Magnetfelderfassungselemente (23), die zuvor von der Ableitungseinheit (76) abgeleitet wurde, oder gemäß einer Intensität des der ersten Wellenform entsprechenden Signals, das zuvor von den mehreren Magnetfelderfassungselementen erfasst wurde, wobei die Erfassungseinheit ferner so konfiguriert ist, dass sie das der ersten Wellenform entsprechende Signal von jedem der Vielzahl von Magnetfelderfassungselementen auf der Grundlage des Synchronisationszeitpunkts erfasst, zu dem das der zweiten Wellenform entsprechende Signal von dem durch die Auswahleinheit ausgewählten Magnetfelderfassungselement erfasst wird.

12. Endoskopsystem nach einem der Ansprüche 1 bis 11, das ferner umfasst: eine Speichereinheit, die so konfiguriert ist, dass sie im Voraus Informationen über das Ansteuerungs-Timing speichert, um das Magnetfeld-Erzeugungselement mit der ersten Wellenform anzusteuern, nachdem sie das Magnetfeld-Erzeugungselement mit der zweiten Wellenform angesteuert hat, wobei die Ableitungseinheit ferner so konfiguriert ist, dass sie eine Position jedes der Vielzahl von Magnetfeld-Erfassungselementen auf der Grundlage der Informationen über das Ansteuerungs-Timing, die in der Speichereinheit gespeichert sind, ableitet.

13. Endoskopsystem nach einem der Ansprüche 1 bis 12, wobei die Magnetfelderzeugungselemente (49), die erste Erzeugungseinheit, die zweite Erzeugungseinheit und die Ansteuerungseinheit nicht elektrisch mit den Magnetfelderfassungselementen (23), der Erfassungseinheit (74, 74A) und der Ableitungseinheit (76) verbunden sind.

14. Endoskopsystem nach Anspruch 12, wobei die Magnetfelderzeugungselemente (49), die erste Erzeugungseinheit (60), die zweite Erzeugungseinheit (62, 62A) und die Ansteuerungseinheit nicht elektrisch mit den Magnetfelderfassungselementen, der Erfassungseinheit, der Ableitungseinheit und der Speichereinheit verbunden sind.

15. Verfahren zur Positionsableitung durch ein Endoskopsystem mit einer Vielzahl von Magnetfelderzeugungselementen (49) und einer Vielzahl von Magnetfelderfassungselementen (23), wobei das Verfahren zur Positionsableitung die folgenden aufeinanderfolgenden Schritte umfasst:
• Erzeugen einer ersten Wellenform zum Erfassen einer Position eines Einführungsteils eines Endoskops, das in ein Subjekt eingeführt werden soll;
• Erzeugen einer zweiten Wellenform zum Erfassen eines Synchronisationszeitpunkts, die sich von der ersten Wellenform in mindestens einer Frequenz, einer Phase, einer Amplitude oder einer Wellenformform unterscheidet und von der ersten Wellenform unterscheidbar ist;
• Ansteuerung der Vielzahl von Magnetfelderzeugungselementen (49) mit der zweiten Wellenform;
• Erfassen einer Synchronisationszeit für jedes der mehreren Magnetfelderzeugungselemente (49), die der Erfassung der zweiten Wellenform durch jedes der mehreren Magnetfelderzeugungselemente (49) entspricht;
• Ansteuern der Vielzahl von Magnetfelderzeugungselementen (49) mit der ersten Wellenform nach dem Ansteuern der Vielzahl von Magnetfelderzeugungselementen (49) mit der zweiten Wellenform;
• Erfassen eines Signals, das der ersten Wellenform entspricht, die von jedem der mehreren Magnetfelderfassungselemente (23) erfasst wird, auf der Grundlage der Synchronisationszeit, die für jedes der mehreren Magnetfelderfassungselemente (23) erfasst wird; und
• Ableiten einer Position jedes der mehreren Magnetfelderfassungselemente (23) auf der Grundlage des erfassten Signals.

## Revendications

1. Un système d'endoscope comprenant :
• une pluralité d'éléments générateurs de champ magnétique (49) ;
• une pluralité d'éléments de détection de champ magnétique (23) ;
• une première unité de génération (60) configurée pour générer une première forme d'onde pour détecter une position d'une partie d'insertion d'un endoscope à insérer dans un sujet ;
• une deuxième unité de génération (62, 62A) configurée pour générer une deuxième forme d'onde, qui diffère de la première forme d'onde par au moins une fréquence, une phase, une amplitude ou une forme d'onde et qui se distingue de la première forme d'onde ;
**caractérisé par le fait que** le système d'endoscope comprend en outre :
• une unité d'entraînement configurée pour entraîner la pluralité d'éléments générateurs de champ magnétique (49) avec la deuxième forme d'onde et configurée en outre pour entraîner la pluralité d'éléments générateurs de champ magnétique avec la première forme d'onde après avoir entraîné la pluralité d'éléments générateurs de champ magnétique (49) avec la deuxième forme d'onde ;
• une unité de détection (72) configurée pour détecter, pour chacun des éléments de détection de champ magnétique, une synchronisation correspondant à la détection de la seconde forme d'onde par chacun des éléments de détection de champ magnétique ;
• une unité d'acquisition (74, 74A) configurée pour acquérir un signal correspondant à la première forme d'onde détectée par chacun des éléments de détection de champ magnétique sur la base de la synchronisation détectée pour chacun des éléments de détection de champ magnétique ; et
• une unité de dérivation configurée pour déduire une position de chacun des éléments de détection de champ magnétique (23) sur la base du signal acquis par l'unité d'acquisition (74, 74A).

2. Système d'endoscope selon la revendication 1, dans lequel l'unité d'entraînement est en outre configurée pour entraîner simultanément chacun des éléments générateurs de champ magnétique (49) avec la deuxième forme d'onde.

3. Système d'endoscope selon la revendication 1, dans lequel la pluralité d'éléments générateurs de champ magnétique (49) sont regroupés en combinaisons de trois éléments générateurs de champ magnétique chacune, et l'unité d'entraînement est en outre configurée pour entraîner simultanément les trois éléments générateurs de champ magnétique dans chaque combinaison avec la même deuxième forme d'onde, la deuxième forme d'onde d'une combinaison se distinguant au moins par la fréquence, la phase, l'amplitude ou la forme d'onde de la deuxième forme d'onde d'une autre combinaison, les combinaisons étant entraînées à un moment différent.

4. Système d'endoscope selon la revendication 1, dans lequel la pluralité d'éléments générateurs de champ magnétique (49) sont regroupés en combinaisons de trois éléments générateurs de champ magnétique chacune, et l'unité d'entraînement est en outre configurée pour entraîner les trois éléments générateurs de champ magnétique dans chaque combinaison avec la même deuxième forme d'onde à un moment différent, la deuxième forme d'onde d'une combinaison se distinguant au moins par la fréquence, la phase, l'amplitude ou la forme d'onde de la deuxième forme d'onde d'une autre combinaison, les combinaisons étant entraînées à un moment différent.

5. Système d'endoscope selon la revendication 1, dans lequel la pluralité d'éléments générateurs de champ magnétique (49) sont regroupés en combinaisons de deux éléments générateurs de champ magnétique ou plus avec différentes orientations de génération de champ magnétique, et l'unité d'entraînement est en outre configurée pour entraîner simultanément les éléments générateurs de champ magnétique dans chaque combinaison avec la même deuxième forme d'onde, la deuxième forme d'onde d'une combinaison se distinguant au moins par la fréquence, la phase, l'amplitude ou la forme d'onde de la deuxième forme d'onde d'une autre combinaison, les combinaisons étant entraînées à une synchronisation différente.

6. Système d'endoscope selon la revendication 1, dans lequel la pluralité d'éléments générateurs de champ magnétique (49) sont regroupés en combinaisons de deux éléments générateurs de champ magnétique ou plus avec différentes orientations de génération de champ magnétique, et l'unité d'entraînement est en outre configurée pour entraîner les éléments générateurs de champ magnétique dans chaque combinaison avec la même deuxième forme d'onde à un moment différent, la deuxième forme d'onde d'une combinaison se distinguant au moins par la fréquence, la phase, l'amplitude ou la forme d'onde de la deuxième forme d'onde d'une autre combinaison, les combinaisons étant entraînées à un moment différent.

7. Système d'endoscope selon la revendication 1, dans lequel l'unité d'entraînement est en outre configurée pour entraîner chacun des éléments générateurs de champ magnétique (49) avec une deuxième forme d'onde qui est différente dans au moins l'un des éléments suivants : une fréquence, une phase, une amplitude ou une forme d'onde, et qui peut être distinguée d'une autre deuxième forme d'onde, chacun des éléments générateurs de champ magnétique (49) étant entraîné à un moment différent.

8. Système d'endoscope selon l'une des revendications 2 à 7, dans lequel les deuxièmes formes d'onde se distinguent les unes des autres par des fréquences différentes ou par des formes d'onde représentant des informations d'identification de l'élément générateur de champ magnétique (49) à piloter.

9. Système d'endoscope selon l'une des revendications 1 à 8, dans lequel l'unité d'acquisition est en outre configurée pour acquérir le signal correspondant à la première forme d'onde détectée par chacun des éléments de détection de champ magnétique (23) sur la base du moment de synchronisation auquel le signal correspondant à la deuxième forme d'onde est détecté par la pluralité d'éléments de détection de champ magnétique (23).

10. Système d'endoscope selon l'une des revendications 1 à 9, comprenant en outre : une unité de sélection configurée pour sélectionner l'élément générateur de champ magnétique (49) à entraîner avec la deuxième forme d'onde, en fonction de la position de chacun de la pluralité d'éléments de détection de champ magnétique (23) précédemment dérivée par l'unité de dérivation (76) ou en fonction d'une intensité du signal correspondant à la première forme d'onde précédemment détectée par la pluralité d'éléments de détection de champ magnétique, l'unité d'entraînement étant en outre configurée pour entraîner l'élément générateur de champ magnétique sélectionné par l'unité de sélection, avec la deuxième forme d'onde.

11. Système d'endoscope selon l'une des revendications 1 à 9, comprenant en outre : une unité de sélection configurée pour sélectionner l'élément de détection de champ magnétique (23) comme cible de détection du signal correspondant à la deuxième forme d'onde, en fonction de la position de chacun de la pluralité d'éléments de détection de champ magnétique (23) précédemment dérivée par l'unité de dérivation (76) ou en fonction d'une intensité du signal correspondant à la première forme d'onde précédemment détectée par la pluralité d'éléments de détection de champ magnétique, L'unité d'acquisition est en outre configurée pour acquérir le signal correspondant à la première forme d'onde à partir de chacun des éléments de détection de champ magnétique sur la base du moment de synchronisation auquel le signal correspondant à la deuxième forme d'onde est détecté par l'élément de détection de champ magnétique sélectionné par l'unité de sélection.

12. Système d'endoscope selon l'une des revendications 1 à 11, comprenant en outre : une unité de stockage configurée pour stocker à l'avance des informations sur la synchronisation de l'entraînement de l'élément générateur de champ magnétique avec la première forme d'onde après l'entraînement de l'élément générateur de champ magnétique avec la deuxième forme d'onde, l'unité de dérivation étant en outre configurée pour dériver une position de chacun de la pluralité d'éléments de détection de champ magnétique sur la base des informations sur la synchronisation de l'entraînement stockées dans l'unité de stockage.

13. Système d'endoscope selon l'une des revendications 1 à 12, dans lequel les éléments générateurs de champ magnétique (49), l'unité de première génération, l'unité de deuxième génération et l'unité d'entraînement ne sont pas connectés électriquement aux éléments de détection de champ magnétique (23), à l'unité d'acquisition (74, 74A) et à l'unité de dérivation (76).

14. Système d'endoscope selon la revendication 12, dans lequel les éléments générateurs de champ magnétique (49), la première unité de génération (60), la deuxième unité de génération (62, 62A) et l'unité d'entraînement ne sont pas connectés électriquement aux éléments de détection de champ magnétique, à l'unité d'acquisition, à l'unité de dérivation et à l'unité de stockage.

15. Méthode de détermination de la position par un système d'endoscope comprenant une pluralité d'éléments générateurs de champ magnétique (49) et une pluralité d'éléments détecteurs de champ magnétique (23), la méthode de détermination de la position comprenant les étapes consécutives suivantes :
• générer une première forme d'onde pour détecter la position d'une partie d'insertion d'un endoscope à insérer dans un sujet ;
• générer une deuxième forme d'onde pour détecter une synchronisation, qui diffère de la première forme d'onde par au moins une fréquence, une phase, une amplitude ou une forme d'onde, et qui se distingue de la première forme d'onde ;
• piloter la pluralité d'éléments générateurs de champ magnétique (49) avec la deuxième forme d'onde ;
• détecter, pour chacun des éléments générateurs de champ magnétique (49), une synchronisation correspondant à la détection de la seconde forme d'onde par chacun des éléments générateurs de champ magnétique (49) ;
• conduire la pluralité d'éléments générateurs de champ magnétique (49) avec la première forme d'onde après avoir conduit la pluralité d'éléments générateurs de champ magnétique (49) avec la seconde forme d'onde ;
• acquérir un signal correspondant à la première forme d'onde détectée par chacun des éléments de détection de champ magnétique (23) sur la base de la synchronisation détectée pour chacun des éléments de détection de champ magnétique (23) ; et
• déterminer la position de chacun des éléments de détection de champ magnétique (23) sur la base du signal acquis.
